(19) Europäisches Patentamt
European Patent Office
Office européen des brevets

(11) Publication number: **0 039 964**
**B1**

(12) # EUROPEAN PATENT SPECIFICATION

(45) Date of publication of patent specification: **14.03.84**

(51) Int. Cl.³: **C 07 C 15/00**, C 07 C 1/04

(21) Application number: **81200432.3**

(22) Date of filing: **15.04.81**

(54) **A process for the preparation of an aromatic hydrocarbon mixture.**

(30) Priority: **06.05.80 NL 8002582**

(43) Date of publication of application:
**18.11.81 Bulletin 81/46**

(45) Publication of the grant of the patent:
**14.03.84 Bulletin 84/11**

(84) Designated Contracting States:
**BE DE GB IT NL**

(56) References cited:
GB - A - 1 555 928
GB - A - 2 037 315
NL - A - 7 803 707
NL - A - 7 811 821

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Boersma, Michael Adriaan Maria**
**Badhuisweg 3**
**NL-1031 CM Amsterdam (NL)**
Inventor: **Post, Martin Franciscus Maria**
**Badhuisweg 3**
**NL-1031 CM Amsterdam (NL)**
Inventor: **Schaper, Lambert**
**Badhuisweg 3**
**NL-1031 CM Amsterdam (NL)**

(74) Representative: **Puister, Antonius Tonnis, Mr. et al,**
**P.O. Box 302**
**NL-2501 CH The Hague (NL)**

**EP 0 039 964 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# A process for the preparation of an aromatic hydrocarbon mixture

The invention relates to a process for the preparation of an aromatic hydrocarbon mixture from a mixture of carbon monoxide and hydrogen using a mixture of two catalysts of which one is capable of catalyzing the conversion of an $H_2/CO$ mixture into acyclic oxygen-containing hydrocarbons and the other is a crystalline silicate capable of catalyzing the conversion of acyclic oxygen-containing hydrocarbons into aromatic hydrocarbons. Said crystalline silicates are characterized in that they have the following properties after calcination for one hour in air at 500°C:

a) an X-ray powder diffraction pattern which comprises as strongest lines, the four lines mentioned in Table A;
b) in the formula which represents the composition of the silicate expressed in moles of the oxides and in which an oxide of a trivalent metal A occurs, in addition to oxides of hydrogen, silicon and optionally of alkali and/or alkaline earth metal, the $SiO_2/A_2O_3$ molar ratio (for the sake of brevity further designated m in this patent application) is 10—100.

TABLE A

| d(Å) | Relative intensity |
|---|---|
| 11.1±0.2 | VS |
| 10.0±0.2 | VS |
| 3.84±0.07 | S |
| 3.72+0.06 | S |

in which the letters used have the following significance:
VS=very strong; S=strong.

During an investigation by the Applicant as regards the above-mentioned process employing catalyst mixtures in which the crystalline silicate component used is an aluminium silicate it was found that both the activity and the selectivity of the catalyst mixtures depend on the value of m of the aluminium silicate in the above-mentioned range. The catalyst mixtures have a high activity and a low selectivity. For application of the process on a commercial scale, a catalyst mixture is required which possesses both a high activity and a high selectivity.

From Netherlands patent application No. 7811821 it is known to use a catalyst mixture containing a crystalline iron silicate in a process for the preparation of a hydrocarbon mixture from an $H_2/CO$ mixture, the silicate having a composition with an $SiO_2/Fe_2O_3$ molar ratio (m) above 10.

It was found that catalyst mixtures which contain a crystalline iron silicate having a value of m of 10—100, possess an activity which is highly dependent on the value of m and a high selectivity which is independent of the value of m. By incorporating a crystalline iron silicate having a value of m of 30—50 into the catalyst mixtures, catalyst mixtures are obtained having a high selectivity and an activity which is comparable with the high activity of a catalyst mixture in which a crystalline aluminium silicate occurs.

The present patent application therefore relates to a process for the preparation of an aromatic hydrocarbon mixture in which a mixture of carbon monoxide and hydrogen is contacted with a mixture of two catalysts one of which is capable of catalyzing the conversion of an $H_2/CO$ mixture into acyclic oxygen-containing hydrocarbons and the other is a crystalline iron silicate which silicate has after calcination for one hour in air at 500°C an X-ray powder diffraction pattern which comprises as strongest lines the four lines mentioned in the following Table

| d(Å) | Relative intensity |
|---|---|
| 11.1±0.2 | VS |
| 10.0±0.2 | VS |
| 3.84±0.07 | S |
| 3.72±0.06 | S |

in which the letters used have the following significance:
VS=very strong; S=strong,

characterized in that in the formula which represents the composition of the silicate expressed in moles of the oxides and in which iron oxide occurs in addition to oxides of hydrogen, silicon and optionally of alkali and/or alkaline earth metal, the $SiO_2/Fe_2O_3$ molar ratio is 30—50.

In the process according to the invention an $H_2/CO$ mixture is used as starting material. Such a

mixture may very suitably be prepared by steam gasification of a carbonaceous material. Examples of such materials are brown coal, anthracite, coke, crude oil and fractions thereof as well as oils derived from tar-sand and bituminous shale. The steam gasification is preferably carried out at a temperature between 900 and 1500°C and a pressure between 10 and 50 bar. In the process according to the invention it is preferred to start from an $H_2/CO$ mixture, the molar ratio of which is between 0.25 and 1.0.

The process according to the invention is preferably carried out at a temperature of between 200 and 500°C and in particular between 300 and 450°C, a pressure of 1—150 bar and in particular of 5—100 bar and a space velocity of 50—5000 and in particular of 300—3000 Nl of gas/l of catalyst/h.

In the process according to the invention a mixture of two catalysts is used which for the sake of simplicity will be designated catalysts X and Y. Catalyst X is the catalyst which is capable of catalyzing the conversion of an $H_2/CO$ mixture into acyclic oxygen-containing hydrocarbons and catalyst Y is the crystalline iron silicate. As catalysts X use is preferably made of catalysts which are capable of converting an $H_2/CO$ mixture into essentially methanol and/or dimethyl ether. Catalysts which contain zinc together with chromium are very suitable for the present purpose. When using a catalyst of this type it is preferred to select a catalyst in which the atomic percentage of zinc based on the sum total of zinc and chromium is at least 60% and in particular 60—80%. It is preferred to use catalyst mixtures which contain 1—5 parts by volume of catalyst X per part by volume of catalyst Y.

The crystalline iron silicate which is present in the catalyst mixtures as catalyst Y is, inter alia, defined with reference to the X-ray powder diffraction pattern. It should contain as strongest lines the four lines mentioned in Table A. The complete X-ray powder diffraction pattern of a typical example of an iron silicate eligible for application according to the invention is shown in Table B:

TABLE B

| d(Å) | Rel. int. | d(Å) | Rel. int. |
|---|---|---|---|
| 11.1 | 100 | 4.00 | 3 |
| 10.0 | 70 | 3.84 | 57 |
| 8.93 | 1 | 3.72 | 31 |
| 7.99 | 1 | 3.64 | 10 |
| 7.42 | 1 | 3.44 | 5 |
| 6.68 | 7 | 3.34 | 3 |
| 6.35 | 11 | 3.30 | 5 |
| 5.97 | 18 | 3.25 | 2 |
| 5.70 | 7 | 3.05 | 5 |
| 5.56 | 10 | 2.98 | 12 |
| 5.35 | 2 | 2.96 | 3 |
| 4.98 | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |

The crystalline iron silicates which are used in the catalyst mixtures can be prepared starting from an aqueous mixture containing the following compounds: one or more compounds which contain an organic cation (R) or from which such a cation is formed during the preparation of the silicate, one or more silicon compounds, one or more iron compounds and optionally one or more compounds of an alkali and/or alkaline earth metal (M). The preparation takes place by maintaining the mixture at elevated temperature until the silicate has been formed and by subsequently separating the silicate crystals from the mother liquor and by washing, drying and calcining the crystals. In the aqueous mixture from which the silicates are prepared the various compounds should be present in the following ratio, expressed in moles of the oxides:

$(M)_{2/n}O:R_2O=0$—20,
$R_2O:SiO_2=0.01$—0.5,
$SiO_2:Fe_2O>30$—100, and
$H_2O:SiO_2=5$—50; n is the valency of M.

During the preparation of the silicates it is preferred to start from a basic mixture in which M is present in a sodium compound and R in a tetrapropyl ammonium compound.

Although the crystalline silicates which are used in the process according to the invention are designated as iron silicates, they may, in addition to iron contain a small quantity of aluminium. The silicon compounds which are eligible from an economic point of view for the preparation of crystalline

silicates on a technical scale, contain as a rule a small quantity of aluminium as impurity. This aluminium is usually found at least in part in the prepared silicate.

The silicates prepared in the above manner may contain alkali and/or alkaline earth metal ions. Using suitable exchange methods they may be replaced by other cations such as hydrogen ions or ammonium ions. The crystalline iron silicates which are used in the catalyst mixtures preferably have an alkali metal content of less than 0.1% by weight and in particular of less than 0.05% by weight. If desired, a binder material, such as bentonite or kaolin may be incorporated in the catalyst mixtures.

The process according to the invention can very suitably be carried out by passing the feed in upward or downward direction through a vertically arranged reactor in which a fixed or moving bed of the relevant catalyst mixtures is present.

The process according to the invention may also very suitably be used as the first stage of a two-stage process for the conversion of an $H_2/CO$ mixture into an aromatic hydrocarbon mixture. In this case at least the $C_2^-$-fraction of the reaction product from the first stage is contacted with a catalyst containing one or more metal components having catalytic activity for the conversion of an $H_2/CO$ mixture into acyclic hydrocarbons, which metal components are selected from the group formed by cobalt, nickel and ruthenium, with the proviso that if the feed for the second stage has an $H_2/CO$ molar ratio of less than 1.5, water is added to this feed and that in the second stage a bifunctional catalyst combination is used which in addition to the metal components with catalytic activity for the conversion of an $H_2/CO$ mixture into acyclic hydrocarbons, also contains one or more metal components with catalytic activity for the conversion of an $H_2O/CO$ mixture into an $H_2/CO_2$ mixture.

The invention will now be elucidated with reference to the following Example.

Example

Six crystalline silicates (silicates 1—6) were prepared by heating mixtures of $SiO_2$, NaOH, $(C_3H_7)_4NOH$ and either $NaAlO_2$ or $Fe(NO_3)_3$ in water in an autoclave at autogenous pressure at 150°C for 24 hours. After cooling of the reaction mixtures the resultant silicates were filtered off, washed with water until the pH of the washing water was approximately 8, dried at 120°C and calcined at 500°C. The silicates 1—6 had the following properties:

a) thermally stable to a temperature above 800°C;
b) an X-ray powder diffraction pattern essentially corresponding to that mentioned in Table B;
c) a value for m as indicated in Table C;

TABLE C

| Silicate no. | $SiO_2/Fe_2O_3$ | $SiO_2/Al_2O_3$ |
|---|---|---|
| 1 | 25 | — |
| 2 | 33 | — |
| 3 | 37 | — |
| 4 | 70 | — |
| 5 | — | 40 |
| 6 | — | 70 |

The molar composition of the aqueous mixtures from which the silicates 1—6 were prepared may be represented as follows:

$$25\ SiO_2 . x\ Fe_2O_3 . y\ Al_2O_3 . 3\ Na_2O . 4.5[(C_3H_7)_4N]_2O . 450\ H_2O$$

wherein x and y have the values shown in Table D:

TABLE D

| Silicate no. | x | y |
|---|---|---|
| 1 | 0.80 | — |
| 2 | 0.66 | — |
| 3 | 0.50 | — |
| 4 | 0.25 | — |
| 5 | — | 0.33 |
| 6 | — | 0.20 |

From the silicates 1—6 the silicates 7—12 were prepared by boiling the silicates 1—6 with 1.0

molar $NH_4NO_3$ solution, washing with water, boiling again with 1.0 molar $NH_4NO_3$ solution and by washing, drying at 120°C and calcining at 500°C. Six catalyst mixtures (catalyst mixtures A—F) were subsequently prepared by mixing a $ZnO—Cr_2O_3$ composition with each of silicates 7—12. The atomic Zn percentage of the $ZnO—Cr_2O_3$ composition was 70%. All catalyst mixtures contained 10 parts by weight of the $ZnO—Cr_2O_3$ composition per part by weight of silicate. The catalyst mixtures A—F were tested for the preparation of an aromatic hydrocarbon mixture from an $H_2/CO$ mixture. Testing was carried out in a 50 ml reactor which contained a fixed catalyst bed having a volume of 7.5 ml. In six experiments an $H_2/CO$ mixture having an $H_2/CO$ molar ratio of 0.5 was passed over each of the catalyst mixtures A—F at a temperature of 375°C, a pressure of 60 bar and a space velocity of 1000 Nl.$1^{-1}$.$h^{-1}$. In all cases a product was obtained, the $C_5^+$ fraction of which consisted for over 30% by weight of aromatics. The other results of the experiments are given in Table E:

TABLE E

| Exp. no. | Catalyst mixture | Silicate no. | Conversion of the synthesis gas after 10 h % | $C_5^+$ selectivity by weight % on $C_1^+$ |
|---|---|---|---|---|
| 1 | A | 7 | 32 | 75 |
| 2 | B | 8 | 65 | 75 |
| 3 | C | 9 | 64 | 75 |
| 4 | D | 10 | 55 | 73 |
| 5 | E | 11 | 66 | 40 |
| 6 | F | 12 | 66 | 45 |

Of the experiments mentioned in Table E only experiments 2 and 3, in which use was made of a catalyst mixture which contained a crystalline iron silicate having an m value between 30 and 50, are experiments according to the invention. The experiments 1 and 4, in which a catalyst mixture was used which contained a crystalline iron silicate having an m value below 30, and above 50 respectively, and the experiments 5 and 6, in which a catalyst mixture was used containing a crystalline aluminium silicate, fall outside the scope of the invention. They have been incorporated for comparison.

It appears from the results mentioned in Table E of the experiments 5 and 6 relating to the conversion of an $H_2/CO$ mixture into an aromatic hydrocarbon mixture using catalyst mixtures which contain a crystalline aluminium silicate having an m value of 10—100 that both the activity and the selectivity of these catalyst mixtures are independent of the m value of the aluminium silicate and also that these catalyst mixtures have a high activity and a low selectivity.

From the results mentioned in Table E of the experiments 1—4 relating to the conversion of an $H_2/CO$ mixture into an aromatic hydrocarbon mixture using catalyst mixtures which contain a crystalline iron silicate having an m value of 10—100, it appears that the selectivity of these catalyst mixtures has a high value which does not depend on m and that the activity of these catalyst mixtures is highly dependent on m and has a high value at an m value of 30—50.

## Claims

1. A process for the preparation of an aromatic hydrocarbon mixture in which a mixture of carbon monoxide and hydrogen is contacted with a mixture of two catalysts one of which is capable of catalyzing the conversion of an $H_2/CO$ mixture into acyclic oxygen-containing hydrocarbons and the other is a crystalline iron silicate which silicate has after calcination for one hour in air at 500°C an X-ray powder diffraction pattern which comprises as strongest lines the four lines mentioned in the following Table

| d(Å) | Relative intensity |
|---|---|
| $11.1 \pm 0.2$ | VS |
| $10.0 \pm 0.2$ | VS |
| $3.84 \pm 0.07$ | S |
| $3.72 \pm 0.06$ | S |

in which the letters used have the following significance:
VS=very strong; S=strong,

characterized in that in the formula which represents the composition of the silicate expressed in moles

of the oxides and in which iron oxide occurs in addition to oxides of hydrogen, silicon and optionally of alkali and/or alkaline earth metal, the $SiO_2/Fe_2O_3$ molar ratio is 30—50.

2. A process as claimed in claim 1, characterized in that the catalyst mixture contains a crystalline silicate having an alkali metal content less than 0.05% by weight.

**Revendications**

1. Un procédé pour la préparation d'un mélange aromatique hydrocarboné dans lequel un mélange de monoxyde de carbure et d'hydrogène est mis en contact avec un mélange de deux catalyseurs, l'un de ceux-ci étant capable de catalyser la conversion d'un mélange $H_2/CO$ en des hydrocarbones acycliques contenant de l'oxygène et l'autre étant un silicate de fer cristallin, ce silicate ayant, après calcination pendant une heure dans l'air à 500°C, un spectre de diffraction en poudre de rayon X qui comprend comme raies les plus fortes les quatre raies mantionnées dans le Tableau suivant:

| d(Å) | Intensité relative |
|---|---|
| 11,1±0,2 | TF |
| 10,0±0,2 | TF |
| 3,84±0,07 | F |
| 3,72±0,06 | F |

dans lequel les lettres utilisées ont la signification suivante:

TF=très forte; F=forte,

caractérisé en ce que, dans la formule qui représente la composition du silicate exprimée en moles d'oxydes et dans laquelle l'oxyde de fer est présent en plus des oxydes d'hydrogène, de silicium et, facultativement, de métal alcalin et/ou alcalino-terreux, le rapport molaire $SiO_2/Fe_2O_3$ est de 30—50.

2. Un procédé selon la revendication 1, caractérisé en ce que le mélange catalyseur contient un silicate cristallin ayant une teneur en métal alcalin inférieure à 0,05 % en poids.

**Patentansprüche**

1. Ein Verfahren zur Herstellung einer Mischung aromatischer Kohlenwasserstoffe, in welchem eine Mischung aus Kohlenmonoxid und Wasserstoff mit einer Mischung aus zwei Katalysatoren kontaktiert wird, von denen einer in der Lage ist, die Umwandlung einer $H_2/CO$-Mischung in acyclische sauerstoffhaltige Kohlenwasserstoffe zu katalysieren, und der andere ein kristallines Eisensilikat ist, welches Silikat nach einstündiger Calcinierung in Luft bei 500°C ein Röntgenpulverdiagramm aufweist, das als stärkste Linien die in der nachstehenden Tabelle angegebenen 4 Linien enthält:

| d(Å) | Relative intensität |
|---|---|
| 11,1±0,2 | VS |
| 10,0±0,2 | VS |
| 3,84±0,07 | S |
| 3,72±0,06 | S |

in welcher die verwendeten Buchstaben die folgende Bedeutung haben:

VS=sehr stark, S=stark,

dadurch gekennzeichnet, daß in der Formel, welche die Zusammensetzung des Silikats wiedergibt, ausgedrückt in Mol der Oxide, und in welcher Eisenoxid zusätzlich zu den Oxiden von Wasserstoff, Silicium und gegebenenfalls Alkali- und/oder Erdalkalimetall vorkommt, das moleare Verhältnis $SiO_2/Fe_2O_3$ 30 bis 50 beträgt.

2. Ein Verfahren wie in Anspruch 1 beansprucht, dadurch gekennzeichnet, daß die Katalysatormischung ein kristallines Silikat mit einem Alkalimetallgehalt von weniger als 0,05 Gew.-% enthält.